Europäisches Patentamt

European Patent Office

Office européen des brevets

(11) Publication number: **0 153 180**
A2

# (12) EUROPEAN PATENT APPLICATION

(21) Application number: **85301059.3**

(22) Date of filing: **18.02.85**

(51) Int. Cl.⁴: **A 61 F 5/44**

(30) Priority: **18.02.84 GB 8404349**
**09.08.84 GB 8420299**

(43) Date of publication of application: **28.08.85**
**Bulletin 85/35**

(84) Designated Contracting States: **AT BE CH DE FR IT LI LU NL SE**

(71) Applicant: **Medical-Assist Limited, Chandlers Row Port Lane, Colchester Essex C01 2JP (GB)**

(72) Inventor: **Wallace, Stephen John, Chandlers Row Port Lane, Colchester Essex C01 2JN (GB)**

(74) Representative: **Green, Alan James et al, Sanderson & Co. 97 High Street, Colchester Essex C01 1TH (GB)**

(54) **Body fluid drainage device, a pack containing the same, and a system based thereon.**

(57) The present invention relates to a body fluid drainage device and system.

The disposable drainage device of the invention comprises a length of flexible tubing having one end sealed within an opening in a drainage bag closed except for the opening accommodating the flexible tubing. The opposite end of the tubing has a connector suitable for connection to the outlet of a leg drainage bag, and the tubing overall has a length e.g. of about 3 feet (91.4 cm), which can provide an overall drainage path from a leg bag attached to a patient in bed to the closed drainage bag positioned outside the bed and below the patient which does not include any loops below the uppermost edge of the drainage bag.

The device of the invention, because it is not connected directly to the bladder, can be packaged in an unsterilised manner. Also, it can form part of a system comprising a leg bag, a device according to the invention, and a floor stand or hanger for the drainage bag.

## BODY FLUID DRAINAGE DEVICE, A PACK CONTAINING
## THE SAME, AND A SYSTEM BASED THEREON

The present invention relates to a body fluid drainage device, a pack containing the same, and a system based thereon. In particular, the invention relates to a urinary drainage device and system.

Artificial drainage of urine from the bladder is necessary when incontinence exists or when the natural process for draining the bladder is impaired or interrupted. Thus, for example, natural drainage may be interrupted in the male by pre-operative retention of urine due to an enlarged prostate or by post-operative impairment of sphincter function due to surgery of prostatic tissue. Similarly, in the female natural drainage may be interrupted after gynaecological surgery.

Artificial drainage can be achieved by introducing a tube (a catheter) into the bladder via the urethra, and the catheter may be withdrawn after emptying of the bladder, or it may be retained and connected to a length of tubing attached to a drainage bag so that urine may be drained continuously. In a post-operative situation, a suitable drainage system is critical to the successful recovery of the patient and, in order to maintain efficient drainage, usually under gravity, the catheter and tubing must both be correctly placed and correctly maintained in position so that there is no impairment of the flow of urine. If the flow of urine is impaired or shut off, retention of urine in the bladder results, with subsequent retrograde invasion of the ureters and kidneys by bacteria passing up from the bladder.

For overnight urine drainage in a patient either with a catheter or an external appliance, it is important that the overall drainage capacity of any system employed should be sufficient to accommodate the amount of urine likely to drain from the patient while asleep. In that situation, generally it has been known to employ one of two systems, namely:
1. For day drainage the semi-ambulant/ambulant patient is placed each morning on a day system comprising a leg bag (or Holster and bag), and each evening the leg bag is removed and the patient is connected for the night to a bedside drainage bag on a stand. This system is an acceptable system in that a leg bag can be worn discreetly, and the patient can be relatively mobile during the day. However, the system involves the serious disadvantage of necessitating the breaking of the closed drainage circuit each morning and each night, with consequent risk of contamination each time the circuit is broken. In that respect, it is to be noted that at least 30% of **all** hospital acquired infections are urinary tract related.

2. Alternatively, the patient may be permanently connected to a bedside drainage bag. This avoids breaking the closed circuit twice daily, but leaves the patient to carry a large bag and stand, if mobility is required, which makes rehabilitation more difficult. Furthermore, use of a night drainage bag during the day acts as a visual reminder to the patient of his or her current condition, and increases psychological trauma, especially in the case of more mobile patients who are ambulant or in wheelchairs.

Recently, it has been proposed to permit patients to continue to wear their day system leg bag at night, and to connect the leg bag outlet rather than the catheter or external appliance to a standard bedside drainage bag. This eliminates the need for disconnecting the leg bag from the catheter or the like with consequent connection of a bedside drainage bag, and thus reduces the likelihood of urinary tract infection due to contamination when the closed drainage circuit is broken.

In the above-mentioned recently proposed system, the leg bag includes a short externally tapered outlet tube from which egress of urine is prevented by an internally tapered cap covering the outlet. At night the leg bag outlet tube cap is removed and the outlet tube is connected to a standard bedside drainage bag using a short length of tubing to act as a female connector between the inlet tubing of the bedside drainage bag and the outlet tube of the leg bag.

However, because the leg bag cannot be completely shut off when the short length of tubing is connected to the leg bag, connection can involve contamination of the hands of the nurse or other operative who puts the system into effect each evening. Also, difficulties can arise out of the use of a standard bedside drainage bag because of the length of inlet tubing attached to such bags, which makes no allowance for the extra length of drainage circuit introduced into the overall system by virtue of the retention of the leg bag.

We have now found that a better system can be provided by basing the system on a disposable night drainage bag rather than a standard bedside drainage bag, the disposable bag being a closed bag i.e. one which includes a length of inlet tubing, but does not include a closeable drainage outlet, and the bag including a length of inlet tubing of an ideal length to accommodate the extra drainage circuit length introduced by the leg bag.

Accordingly, the present invention provides a disposable body fluid drainage device, which device comprises a length of flexible tubing having one end sealed within an opening in a drainage bag closed except for the opening accommodating the flexible tubing, and having at its opposite end a connector suitable for connection to the outlet of a leg drainage bag, the tubing having a length which can provide an overall drainage path from a leg bag attached to a patient in bed to the closed drainage bag positioned outside the bed and below the patient which does not include any loops below the uppermost edge of the bag.

In the device of the invention, the connector is preferably a flexible connector, more preferably a flexible female connector which can be fitted over an outlet tube arrangement of a leg bag to form a fluid-tight connection, whether that arrangement is a capped tube arrangement as in the above-mentioned prior art system, or more preferably an arrangement including a cylindrical valve as in the system of the present invention described below. The female connector is preferably a short length of flexible tubing, preferably silicone rubber tubing.

In order to accommodate the volume of urine excreted overnight, the closed drainage bag preferably has a capacity of from about 1 litre to about 2 litres, more preferably about 2 litres. Preferably also, the closed drainage bag includes means to permit opening by tearing, and thus without contamination of scissors or the like. For example, the closed drainage bag may be essentially rectangular, and may have a line of weakness in a corner nearest the flexible tubing i.e. a corner which is uppermost in use, to permit opening of the bag by tearing, and consequent emptying thereof before disposal.

Preferably, the flexible tubing of the device of the invention is directly connected to the inside of the drainage bag, that is to say there are no intervening non-return valves, chambers or other like devices. Furthermore, since a bedside drainage bag ideally has an inlet tubing length of about 4 feet (1.22 metres) - although some have a tubing length of 1 metre - and since a leg bag provides a drainage path length of about 1 foot (30.48 cm), the flexible tubing of the device of the invention preferably has a length of about 3 feet (91.4 cm), so that when the bag is placed on a floor stand or hanger the extra length of drainage circuit presented by the leg bag is accommodated.

In addition, it is preferred that the flexible tubing should be welded to the bag within the bag opening. Thus, preferably the tube and bag are of materials which can be welded together to form a unitary structure.

The device of the invention generally will be put up for use packaged in an unsterilised manner. Typically, a use pack will comprise a multiplicity of devices according to the invention, each packaged in an unsterilised manner, and preferably such a pack will comprise one week's supply i.e. seven said devices.

As will be appreciated, the fact that the present device can be presented without sterilisation is an advantage in terms of cost saving. Furthermore, there is no risk to the patient because the device is never directly connected to the patient via a catheter or the like.

The invention also includes a body fluid drainage system, which system comprises a leg bag including an outlet valve having an outer cylindrical configuration and a device according to the invention in which the connector is adapted to fit over the outlet valve of the leg bag to form a fluid-tight connection.

In such a drainage system according to the invention there is preferably included a floor stand or hanger for the drainage bag. Preferably also the stand or hanger includes a spiral element e.g. a pigtail, within which the flexible tubing can be threaded to maintain it in an unobstructed disposition throughout a night, i.e. disposed in a manner so as not to impair urine flow.

In the system of the invention the outlet valve may be any valve (or tap) arrangement of suitable size. However, it is preferably a valve as described specifically below where the outer cylindrical surface carries a multiplicity of splines.

The device and system of the present invention, in common with other medical appliances, can pose a problem of contamination after use. Furthermore, while in a hospital context there may be procedures for safe handling and disposal of contaminated materials, when appliances are used at home, such procedures are invariably absent and the problem may be compounded. In addition, drained fluids containing bacteria or other organisms will permit the colonies of organisms (which may be pathogenic) to increase in number in direct relation to the length of time which elapses prior to emptying and disposal.

In the home, contamination of medical appliances generally presents two main hazards. First, to the person, usually other than the patient, emptying the appliance; and secondly to refuse collectors, and even to members of the general public, who otherwise may later come into contact with an emptied appliance containing residual contaminated fluid. In the latter case, the hazard involved can be increased by virtue of the fact that the emptied appliance will have continued subsequent to emptying to compound its colony of bacteria or other organisms.

In order to overcome the above-mentioned problem, the drainage bag of the device of the invention preferably includes a disinfectant compound or composition, generally in a disinfecting amount. Such a disinfectant is preferably in solid form, and moreover is preferably incorporated in the bag during manufacture or assembly of the device. More preferably, the disinfectant is one which has a vapour pressure sufficient to disinfect the flexible tubing connected to the bag.

While a wide range of disinfectants may be employed within the above criteria, by way of example

- 6 -                          0153180

only, the following commercially-available substances are mentioned as suitable disinfectants, namely:

Chlorhexidine,
Noxythiolin,
Paraformaldehyde,
Hyperchlorite, and
Sodium dichloro-s-triazinetrione,

all of which may be provided in solid form.

Since the device of the invention is not, when in use, directly connected to the patient's urinary tract, reflux of toxic and/or irritant disinfectant cannot take place, and the disinfectant employed need not be nontoxic and/or non-irritant.

The invention will now be described by way of example with reference to the accompanying drawings in which:

Figure 1 shows one form of a device according to the invention;

Figures 2(a) and 2(b) show alternative leg bag outlet tubes, and

Figure 3 shows a drainage bag hanger.

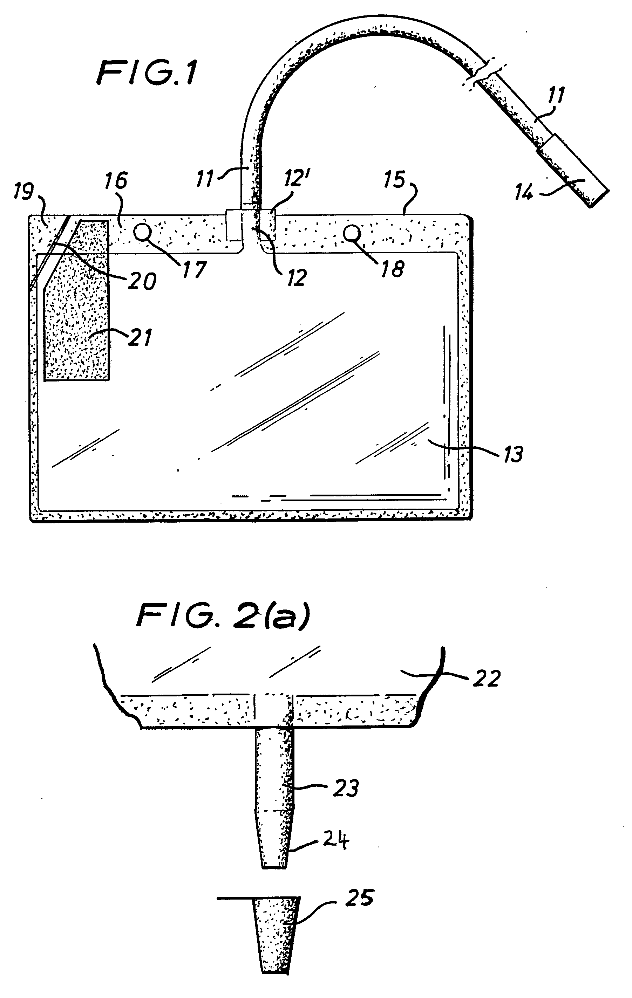

Referring to Figure 1, the device shown comprises a length, preferably approximately 3 ft (91.4 cm), of flexible tubing 11, having one end welded within an opening 12 of a drainage bag 13 closed except for the opening 12, so as to form a unitary structure 12' around the opening 12. At its opposite end the tubing 11 has a flexible female connector 14 which can be fitted over an outlet tube arrangement of a leg bag to form a fluid-tight connection, and which comprises a short length of silicone rubber tubing.

The drainage bag 13 is of 2 litre capacity and comprises opaque or semi-opaque or translucent plastics sheet material heat-sealed around its four edges to form a bag. The bag is of generally rectangular configuration, and at its upper edge 15 includes a flap portion 16 having holes accommodating studs 17 and 18 to permit the bag to be hung from a drainage bag hanger with the tubing 11 uppermost. The tubing 11 feeds directly into the uppermost portion of the bag without any intervening non-return or other valve arrangements. Also, as will be seen, the bag includes no outlet valve or other means to permit its re-use.

At an upper corner 19, the bag 13 includes a line of weakness 20 to permit the opening and emptying of the bag by tearing before disposal thereof. Once opened by tearing away corner 19, the bag 13 cannot be safely reused. Adjacent corner 19 there is a panel 21 of use instructions.

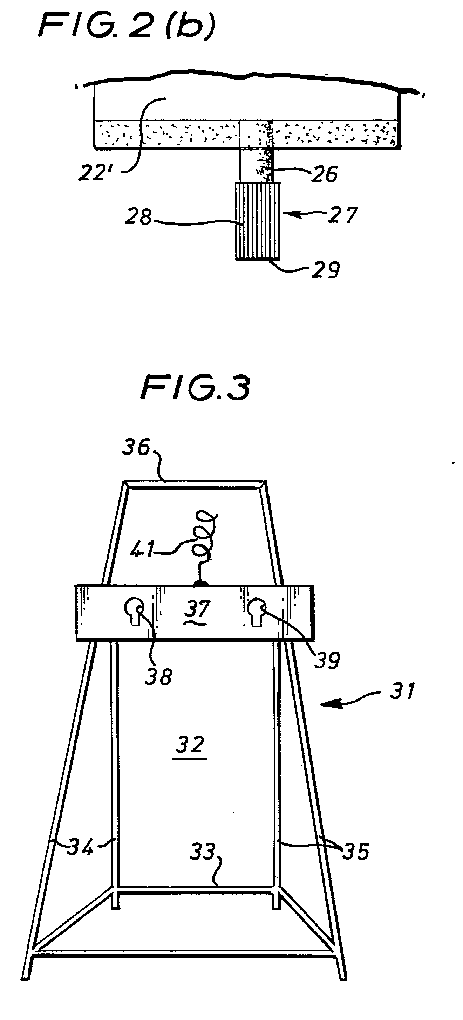

Referring to Figures 2(a) and 2(b), those show the bottom outlet drainage tube arrangements of two different leg bags. In the arrangement of Figure 2(a), a leg bag 22 includes an outlet tube 23 having a lower conical portion 24 which can be closed by a complementary conical cap 25. With the cap 25 removed the connector 14 can be push-fitted over the conical portion 24 to form a fluid-tight overnight connection.

Similarly, as shown in Figure 2(b), a leg bag 22' includes an outlet tube 26 having a valve arrangement 27 including an outer cylindrical surface 28 carrying a multiplicity of splines 29. Normally the splines 29 are provided to enable the nurse or other operative to grip the valve to permit its opening and closure. However, in the system of the present invention the splines provide a convenient means of ensuring that the connection between the connector 14 and tube 26 while safe is also not too tenacious, so disconnection is not rendered difficult due to the connector sticking to a smooth outer surface. Furthermore, the valve 27 provides for the avoidance of hand contamination during connection and disconnection, and is available as needed to permit drainage from the leg bag 22' into bag 13.

Referring to Figure 3, that shows a drainage bag stand 31 comprising a plastics coated frame 32 welded from steel wire rods and comprising a rectangular base portion 33 including two pairs of upstanding arms 34 and 35 forming inverted 'V' shaped sides to the stand. At the top of the stand there is a handle 36, and on one face of the frame 32 there is a plate 37 including keyhole slots 38 and 39 from which a drainage bag can be suspended using studs 17 and 18. Upstanding from the top centre of the plate 37 there is a pigtail support 41 to accommodate the tubing 11.

In use, when the patient is ready to retire, the device shown is connected via connector 14 to the outlet tube 23 or 26 of the patient's leg bag, and valve 27 (if present) is opened. The bag 13 is then hung on a stand 31 using studs 17 and 18 to connect the bag to the keyhole slots 38 and 39. In the morning, valve 27 (if present) is closed, and the connector 14 is then removed from the drainage bag outlet tube 23 or 26. For disposal, corner 19 is torn away, the bag is emptied into a drainage sluice, and disposed of in the normal way.

Since the arrangement shown in Figure 2(b) permits of a dry or relatively dry connection and disconnection of the connector 14, in the system of the invention as described above and as defined below, the leg bag is one which includes a valve arrangement essentially as shown.

It will be appreciated from the above specific description that the independence night bag extension drainage system of the present invention can provide a complete system in the management of patient well-being and infection control which offers the following advantageous features:

1.   The closed circuit between the catheter or the like and day leg bag remains undisturbed.

2.   Hand contamination is reduced by the preferred connection.

3.   Use of a leg bag or Holster is encouraged for many more patients, which will ease the psychological trauma associated with the use of a night drainage bag during the day.

4.   Rehabilitation is assisted by removing the encumbrance of a large bed drainage bag.

5.   Teaching to learner nurses and all carers involved e.g. relatives, home helps, community nurses etc., is simplified.

6.   Safer catheter drainage in patients' own homes, nursing homes and Part III accommodation is promoted.

7.   Self care and independence is encouraged; patients are more likely to be able to cope on their own, giving a beneficial psychological effect.

8.   Dispensing by the family doctor is made simple by the use of a 7-day pack system.

In addition, the system is cost effective in terms of both time and equipment. Thus, for example, the device of the invention is much less expensive to produce than a standard drainage bag since it includes no outlet tap or non-return valve arrangements, and the patient's leg bag can remain in situ for a relatively long period of time e.g. seven days, and need not be changed each day.

In addition, by using the system, a nightly visit say by a community nurse to change a patient from a day bag to a night bag is no longer necessary. Furthermore, the system may well enable a leg bag now to be used in situations where previously patients were forced to use an inappropriate bed drainage bag all the time, because they had no facility to cope with the breaking of the drainage circuit.

## CLAIMS

1. A disposable body fluid drainage device, which device comprises a length of flexible tubing having one end sealed within an opening in a drainage bag closed except for the opening accommodating the flexible tubing, and having at its opposite end a connector suitable for connection to the outlet of a leg drainage bag, the tubing having a length which can provide an overall drainage path from a leg bag attached to a patient in bed to the closed drainage bag positioned outside the bed and below the patient which does not include any loops below the uppermost edge of the drainage bag.

2. A device according to claim 1, wherein the connector is a flexible female connector which can be fitted over an outlet tube arrangement of a leg bag to form a fluid-tight connection, preferably a female connector comprising a short length of flexible tubing, for example, one comprising silicone rubber tubing.

3. A device according to claim 1 or claim 2, which includes means to permit opening of the closed drainage bag by tearing, and preferably wherein the closed drainage bag has a line of weakness in a corner nearest the flexible tubing to permit opening of the bag by tearing, and emptying thereof before disposal.

4. A device according to any one of the preceding claims, wherein the flexible tubing is directly connected to the inside of the drainage bag.

5. A device according to any one of the preceding claims, wherein the drainage bag includes a disinfecting amount of a disinfectant compound or composition.

6. A device according to claim 5, wherein the disinfectant is provided in solid form.

7. A device according to claim 5 or claim 6, wherein the disinfectant is one which has a vapour pressure sufficient to disinfect the flexible tubing connected to the bag.

8. A device according to any one of claims 5 to 7, wherein the disinfectant is one or more of chlorhexidine, noxythiolin, paraformaldehyde, hyperchlorite and sodium dichloro-s-triazinetrione.

9. A device according to any one of the preceding claims when packaged in an unsterilised manner.

0153180

10. A pack comprising a multiplicity of devices according to claim 9, e.g. seven said devices, each packaged in an unsterilised manner.

11. A body fluid drainage system, which system comprises a leg bag including an outlet valve arrangement having an outer cylindrical configuration, preferably a valve wherein the outer cylindrical surface of the valve carries a multiplicity of splines, and a device according to any one of claims 1 to 8 in which the connector is adapted to fit over the outlet valve arrangement of the leg bag to form a fluid-tight connection.

12. A system according to claim 11, including a floor stand or hanger for the drainage bag, preferably a stand or hanger including a spiral element within which the flexible tubing can be threaded to maintain it in an unobstructed disposition throughout a night.

# FIG.1

# FIG. 2(a)

0153180

## FIG. 2 (b)

## FIG.3